(19) Europäisches Patentamt
European Patent Office
Office européen des brevets

(11) Veröffentlichungsnummer: **0 155 587**
**B1**

# (12) EUROPÄISCHE PATENTSCHRIFT

(45) Veröffentlichungstag der Patentschrift: **08.06.88**

(51) Int. Cl.[4]: **C 07 D 471/04**, C 07 D 215/58, A 61 K 31/47, A 61 K 31/44

(21) Anmeldenummer: **85102447.1**

(22) Anmeldetag: **05.03.85**

## (54) 1,7-Diamino-1,4-dihydro-4-oxo-3-(aza) chinolincarbonsäuren, Verfahren zu ihrer Herstellung sowie ihre Verwendung bei der Bekämpfung bakterieller Erkrankungen.

(30) Priorität: **17.03.84 DE 3409922**

(43) Veröffentlichungstag der Anmeldung: **25.09.85 Patentblatt 85/39**

(45) Bekanntmachung des Hinweises auf die Patenterteilung: **08.06.88 Patentblatt 88/23**

(84) Benannte Vertragsstaaten: **AT BE CH DE FR GB IT LI NL SE**

(56) Entgegenhaltungen:
**EP-A-0 009 425**
**EP-A-0 027 752**
**EP-A-0 090 424**
**EP-A-0 126 355**
**GB-A-2 094 305**
**US-A-4 284 629**
**US-A-4 398 029**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Grohe, Klaus, Dr., Am Wasserturm 10, D-5068 Odenthal (DE)**
Erfinder: **Zeiler, Hans- Joachim, Dr., Elsbeeker Strasse 46, D-5620 Velbert 15 (DE)**
Erfinder: **Metzger, Karl- Georg, Dr., Pahlkestrasse 75, D-5600 Wuppertal 1 (DE)**

**EP 0 155 587 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft neue 1,7-Diamino-1,4-dihydro-4-oxo-3-(aza)chinolincarbonsäuren, Verfahren zu ihrer Hestellung sowie ihre Verwendung bei der Bekämpfung bakterieller Erkrankungen.

Es wurde gefunden, daß die neuen 1,7-Diamino-1,4-dihydro-4-oxo-3-(aza)chinolincarbonsäuren der allgemeinen Formel I

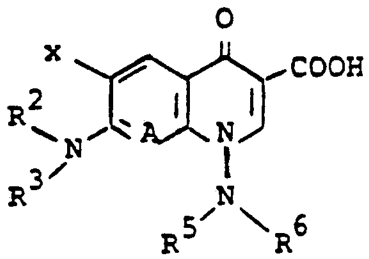

(I)

in der

A entweder Stickstoff oder C-$R^1$ bedeutet, wobei $R^1$ für die Nitrogruppe oder Halogen, bevorzugt Fluor steht,

$R^2$ und $R^3$ gleich oder verschieden sein können und für einen $C_1$-$C_3$-Alkylrest stehen und weiterhin gemeinsam mit dem Stickstoffatom, an das sie gebunden sind, einen 5- oder 6-gliedrigen heterocyclischen Ring bilden können, der als Ringglied zusätzlich die Atome oder Gruppen -O-, -S-, -SO-, -$SO_2$- oder -N-$R^4$ enthalten kann und der gegebenenfalls an den Kohlenstoffatomen ein- bis dreifach durch $C_1$-$C_3$-Alkyl, Hydroxy, Alkoxy mit 1-3 Kohlenstoffatomen, Amino, Methylamino oder Ethylamino substituiert sein kann, wobei jeweils ein Kohlenstoffatom nur einen Substituenten tragen kann, wobei

$R^4$ für Wasserstoff, eine verzweigte oder unverzweigte Alkyl- oder Alkenylgruppe mit 1 bis 4 Kohlenstoffatomen, die gegebenenfalls durch eine Hydroxy-, Alkoxy-, Alkylmerkapto-, Alkylamino- oder Dialkylamino-gruppe mit 1 bis 3 Kohlenstoffatomen für einen Alkylrest, die Cyangruppe, die Alkoxycarbonylgruppe mit 1 bis 4 Kohlenstoffatomen im Alkoholteil substituiert sein kann, die Benzyloxycarbonylgruppe, eine gegebenenfalls im Phenylrest substituierte Phenylalkylgruppe mit bis zu 4 Kohlenstoffatomen im aliphatischen Teil, einen gegebenenfalls durch Hydroxy, Methoxy, Chlor und Fluor ein- oder zweifach substituierten Phenylrest, einen Oxoalkylrest mit bis zu 6 Kohlenstoffatomen sowie einen Cycloalkyl-alkylrest mit bis zu 6 Kohlenstoffatomen im cyclischen Teil und bis zu 3 Kohlenstoffatomen im acyclischen Teil steht, sowie

$R^5$ und $R^6$ gleich oder verschieden sein können und Wasserstoff oder eine Alkylgruppe mit 1 bis 4 Kohlenstoffatomen bedeuten und ferner

X für Wasserstoff, die Nitrogruppe oder Halogen, bevorzugt Fluor oder Chlor steht, und deren pharmazeutisch verwendbare Salze,

sich als antibakterielle Wirkstoffe für die Human- und Veterinärmedizin, wobei zur Veterinärmedizin auch die Vorbeugung und Behandlung bei Fischen zu zählen ist, eignen.

Bevorzugt sind diejenigen Verbindungen der allgemeinen Formel I, in denen
X und $R^1$ Halogen, bevorzugt Fluor oder Chlor, darstellen und
$R^2$, $R^3$, $R^4$, $R^5$ und $R^6$ die oben angegebene Bedeutung besitzen.

Besonders bevorzugt sind diejenigen Verbindungen der allgemeinen Formel I in denen
A entweder Stickstoff oder C-$R^1$ bedeutet, wobei
$R^1$ für Nitrogruppe steht,
X, $R^2$, $R^3$, $R^4$, $R^5$ und $R^6$ die oben angegebene Bedeutung besitzen.

Weiterhin wurde gefunden, daß man die 1,7-Diamino-1,4-dihydro-4-oxo-3-(aza)chinolincarbonsäuren der allgemeinen Formel I erhält, wenn man die 1-Amino-3-(aza)-chinolincarbonsäuren der allgemeinen Formel II

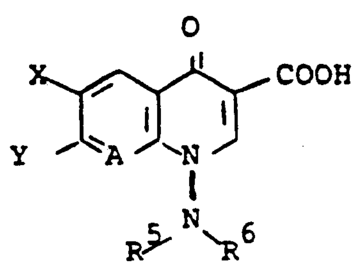

(II)

in der
X, A und $R^5$, $R^6$ die oben angegebene Bedeutung haben
und
Y für Halogen, bevorzugt Chlor oder Fluor, steht,
mit Aminen der allgemeinen Formel III

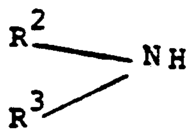

(III)

in welcher
$R^2$ und $R^3$ die oben angegebene Bedeutung haben,
gegebenenfalls in Gegenwart von Säurebindern umsetzt.

Verwendet man bei der Umsetzung Piperazin und 1-Amino-6,7,8-trifluor-1,4-dihydro-4-oxo-3-chinolincarbonsäure als Augsgangsstoffe, so kann der Reaktionsablauf durch das folgende Formelschema wiedergegeben werden:

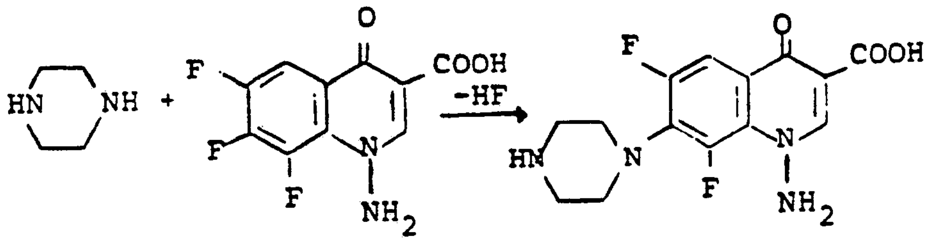


Die als Ausgangsstoffe verwendbaren 1-Amino-3-(aza)-chinolincarbonsäuren der allgemeinen Formel II können gemäß folgendem Reaktionsschema hergestellt werden:

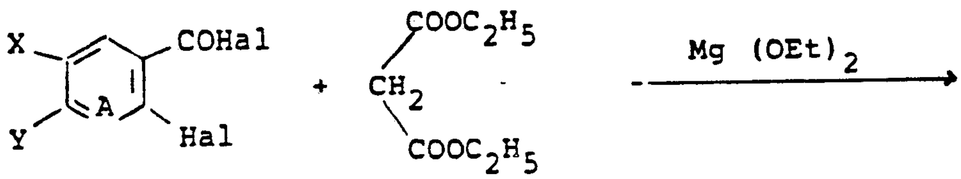


(1) (2)

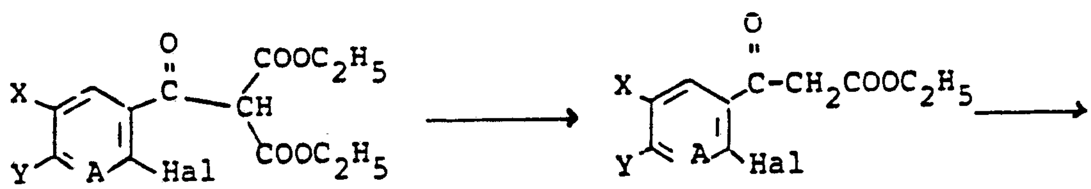

(3) (4)

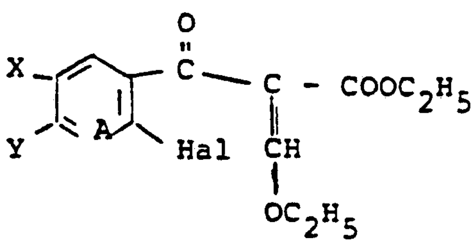

(5)

Danach wird Malonsäurediethylester (2) in Gegenwart von Magnesiumethylat mit den o-Halogenaroylhalogeniden (1) zu den Aroylmalonestern (3) acyliert (Organicum, 3. Aufl. 1964, S. 438). Durch partielle Verseifung und Decarboxylierung von (3) in wäßrigem Medium mit katalytischen Mengen Schwefelsäure oder p-Toluolsulfonsäure erhält man in guter Ausbeute die Aroylessigsäureethylester (4), die mit o-Ameisensäure-triethylester/Acetanhydrid in die 2-Aroyl-3-ethoxy-acrylsäureethylester (5) übergehen.

a) Die Umsetzung von (5) mit 1,1-Dialkylhydrazinen (6) ($R^5$, $R^6$ = $C_1$-$C_4$-Alkyl) führt zu den 2-Aroyl-3-hydrazino-acrylsäureestern (7). Die exotherme Reaktion wird in einem Lösungsmittel wie z. B. Ethanol, Methylenchlorid oder Toluol durchgeführt.

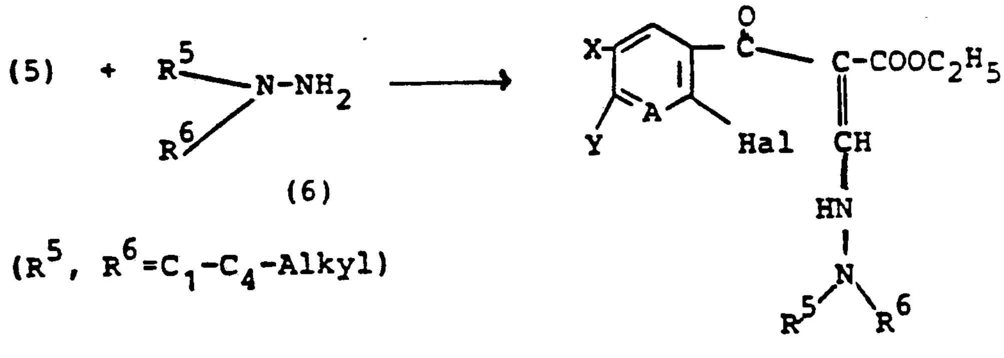

(6)

$(R^5, R^6 = C_1\text{-}C_4\text{-Alkyl})$

(7)

b) Mit 1-Alkyl-1-acyl-hydrazinen (8) liefert (5) die 2-Aroyl-3-hydrazino-acrylsäureester (9).

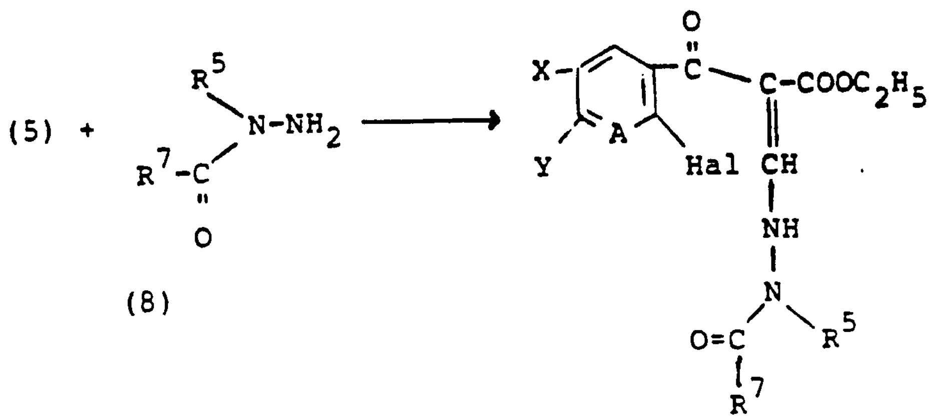

(8)

(9)

Dabei steht für $R^5$ $C_1$-$C_4$-Alkyl und für $R^7$ Wasserstoff, Alkyl mit 1-3 Kohlenstoffatomen, Phenyl und Alkoxy mit 1-3 Kohlenstoffatomen.

c) Und schließlich gehen die Hydrazone (10) mit (5) in die Enhydrazine (11) über.

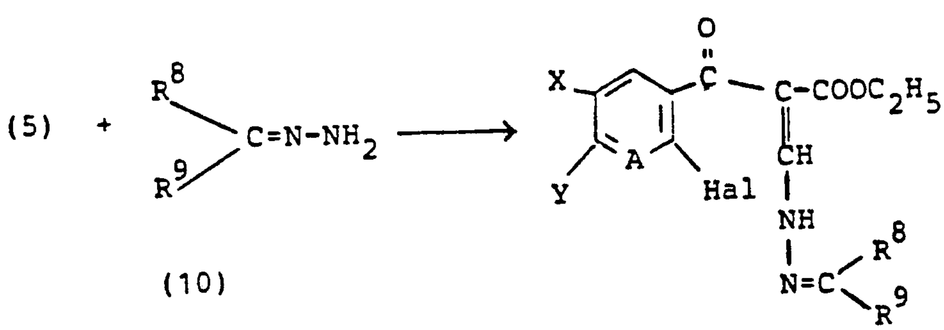

(10)

(11)

$R^8$ kann Wasserstoff, $C_1$-$C_3$-Alkyl oder Phenyl

$R^9$ $C_1$-$C_3$-Alkyl oder Phenyl bedeuten.

Die 2-Aroyl-3-hydrazino-acrylsäureester (7), (9) und (11) werden gegebenenfalls in Gegenwart eines Verdünnungsmittels und eines Säurebinders in einem Temperaturbereich von etwa 60-300°C, bevorzugt 80-180°, zu den substituierten 1-Amino-3-(aza)chinoloncarbonsäureestern (12), (13) bzw. (14) cyclisiert.

(7)

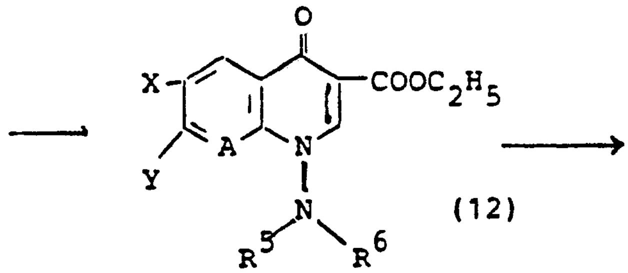

(12)

(II)

(9)

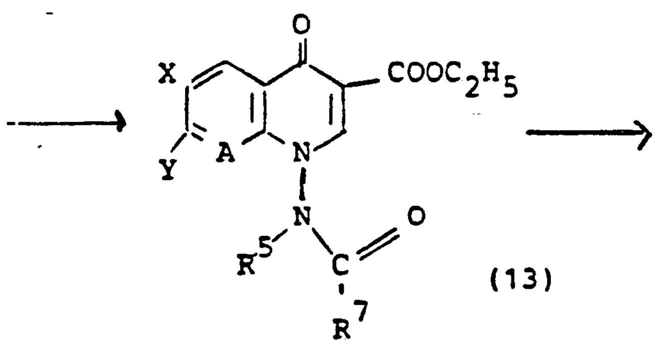

(13)

(II)

(11)

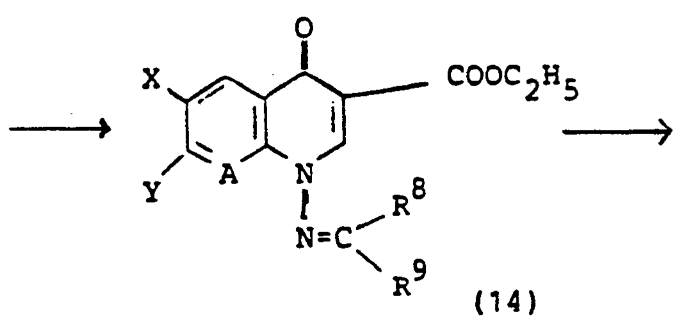

(14)

(II)

Als Verdünnungsmittel können Toluol, Dioxan, Dimethylsulfoxid, N-Methylpyrrolidon, Sulfolan, Hexamethylphosphorsäuretriamid und N,N-Dimethylformamid verwendet werden.

Als Säurebinder kommen für diese Reaktionsstufe Kalium-tert.-butanolat, Butyl-lithium, Lithium-phenyl, Natrium-methylat, Natriumhydrid, Natrium- oder Kaliumcarbonat, DBU (1,8-Diazabicyclo [5.4.0]' undec-7-en)

und falls das Halogenatom in 2-Stellung des Aroylrestes von (7), (9) oder (11) Fluor ist, bevorzugt Kalium- oder Natriumfluorid in Betracht. Es kann vorteilhaft sein, einen Oberschuß von 5 Mol-% an Base einzusetzen.

Die im letzten Schritt erfolgende Esterhydrolyse von (12) unter basischen oder sauren Bedingungen führt zu den 3-(Aza)chinoloncarbonsäuren (II) ($R^5$, $R^6$ ist $C_1$-$C_4$-Alkyl).

Bei der Umsetzung der 1-Acylamino-3-(aza)-chinoloncarbonsäurreester (13) mit 2 Äquivalenten Alkalilauge wird der Acylrest abgespalten und die Estergruppe verseift. Man erhält die entsprechenden 3-(Aza)chinoloncarbonsäuren II ($R^5$ = H, $R^6$ = $C_1$-$C_4$-Alkyl) in guter Ausbeute.

Stellen die Reste $R^8$ und $R^9$ der Hydrazonester (14) beispielsweise eine Methylgruppe dar, so kann die Spaltung zu den 1-Amino-3-(aza)chinoloncarbonsäurreestern und Aceton in Gegenwart von 1 Mol $H_2O$ und katalytischen Mengen p-Toluolsulfonsäure erfolgen. Das Aceton wird dabei mit Isopropanol aus dem Gleichgewicht destilliert. Die 1-Amino-3-(aza)chinoloncarbonsäureester werden dann alkalisch zu den entsprechenden 3-(Aza)chinoloncarbonsäuen II ($R^5$=$R^6$=H) verseift.

Die als Ausgangsstoffe verwendeten o-Halogen-(het) aroylhalogenide (1) sind bekannt oder können nach literaturbekannten Verfahren erhalten werden. Beispielhaft sei genannt: 2,6-Dichlor-nicotinsäurechlorid [F. Mutterer und C. D. Weis, Helv. Chim. Acta 59, 222 (1976)].

2,3,4,5-Tetrafluorbenzoylchlorid bzw. 2,4,5-Trifluorbenzoylchlorid wurden aus den literaturbekannten 2,3,4,5-Tetrafluor-benzoesäure [G.G. Yakobsen, V.N. Odinokov und N.N. Vorozhtsov Jr. , Zh. Obsh. Khim. 36, 139 (1966)] und 2,4,5-Trifluor-benzoesäure [J.I. DeGraw, M. Cory) und W.A. Skinner, J. Chem. Eng. Data 13, 587 (1968) mit Thionylchlorid auf übliche Weise erhalten. Das 2,3,4,5-Tetrafluorbenzoylchlorid besitzt einen Siedepunkt von 75-80° C/17 mbar. Das 2,4,5-Trifluorbenzoylchlorid einen solchen von 82-85° C/13 mbar.

Das 2,4-Dichlor-5-fluor-3-nitro-benzoylchlorid wurde durch Nitrierung der 2,4-Dichlor-5-fluor-benzoesäure zur 2,4-Dichlor-5-fluor-3-nitrobenzoesäure und deren Umsetzung mit Thionylchlorid erhalten.

Die als Ausgangsstoffe verwendeten Amine III sind bekannt oder können nach literaturbekannten Verfahren erhalten werden. Als Beispiele seien genannt:

Piperidin, Morpholin, Pyrrolidin, Dimethylamin, Piperazin, 1-Methylpiperazin, 1-Ethylpiperazin, 1-β-Hydroxyethylpiperazin, 1-Formylpiperazin, 2-Methylpiperazin, 1,2-Dimethylpiperazin, 2-Ethylpiperazin, 2-Propylpiperazin.

Die als Ausgangsstoffe verwendeten Hydrazinderivate (6), (8) bzw. (10) sind ebenfalls bekannt oder können nach literaturbekannten Verfahren hergestellt werden.

Als Beispiele seien genannt:

Dimethylhydrazin, Diethylhydrazin, Di-n-butylhydrazin, 1-Formyl-1-methylhydrazin, 1-Formyl-1-ethylhydrazin, 1-Formyl-1-n-butylhydrazin, 1-Acetyl-1-methylhydrazin, 1-Acetyl-1-ethylhydrazin, 1-Benzoyl-1-methylhydrazin, 1-Ethoxycarbonyl-1-methylhydrazin, Aceton-hydrazon, Benzaldehyd-hydrazon, Acetophenon-hydrazon, Benzophenon-hydrazon, Methylethylketon-hydrazon.

Die Umsetzung von II mit III wird vorzugsweise in einem Verdünnungsmittel wie Dimethylsulfoxid, N,N-Dimethylformamid, Hexamethylphosphorsäuretrisamid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindemittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2,2,2]-octan (DABCO), überschüssiges Amin III oder 1,8-Diazabicyclo[5,4,0]undec-7-en (DBU).

Die Reaktionstemperaturen können in einem größeren Bereich variiert werden. Im allgemeinen arbeitet man zwischen etwa 0 und 200° C, vorzugsweise zwischen 20 und 160° C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens setzt man auf 1 Mol der Carbonsäure II 1 bis 15 Mol, vorzugsweise 1 bis 6 Mol des Amins III ein.

Als neue Wirkstoffe seien im einzelnen genannt:

1-Amino-6,8-difluor-1,4-dihydro-4-oxo-7-(1-piperazinyl)-3-chinolincarbonsäure,
1-Amino-6,8-difluor-1,4-dihydro-4-oxo-7-(4-methyl-1-piperazinyl)-3-chinolincarbonsäure,
1-Amino-6,8-difluor-1,4-dihydro-4-oxo-7-(4-ethyl-1-piperazinyl)-3-chinolincarbonsäure,
1-Amino-6,8-difluor-1,4-dihydro-4-oxo-7-(3-methyl-1-piperazinyl)-3-chinolincarbonsäure,
1-Amino-6,8-difluor-1,4-dihydro-4-oxo-(3,4-dimethyl-1-piperazinyl)-3-chinolincarbonsäure,
1-Methylamino-6,8-difluor-1,4-dihydro-4-oxo-7-(1-piperazinyl)-3-chinolincarbonsäure,
1-Methylamino-6,8-difluor-1,4-dihydro-4-oxo-7-(4-methyl-1-piperazinyl)-3-chinolincarbonsäure,
1-Methylamino-6,8-difluor-1,4-dihydro-4-oxo-7-(4-ethyl-1-piperazinyl)-3-chinolincarbonsäure,
1-Dimethylamino-6,8-difluor-1,4-dihydro-4-oxo-7-(1-piperazinyl)-3-chinolincarbonsäure,
1-Dimethylamino-6,8-difluor-1,4-dihydro-4-oxo-7-(4-methyl-1-piperazinyl)-3-chinolincarbonsäure,
1-Dimethylamino-6-fluor-8-nitro-1,4-dihydro-4-oxo-7-(1-pyrrolidinyl)-3-chinolincarbonsäure,
1-Dimethylamino-6-fluor-8-nitro-1,4-dihydro-4-oxo-7-(1-piperazinyl)-3-chinolincarbonsäure,
1-Dimethylamino-6-fluor-8-nitro-1,4-dihydro-4-oxo-7-(4-methyl-1-piperazinyl)-3-chinolincarbonsäure,
1-Methylamino-6-fluor-8-nitro-1,4-dihydro-4-oxo-7-(1-piperazinyl)-3-chinolincarbonsäure,
1-Methylamino-6-fluor-8-nitro-1,4-dihydro-4-oxo-7-(4-methyl-1-piperazinyl)-3-chinolincarbonsäure,
1-Amino-6-fluor-8-nitro-1,4-dihydro-4-oxo-7-(1-piperazinyl)-3-chinolincarbonsäure,

1-Amino-6-fluor-8-nitro-1,4-dihydro-4-oxo-7-(4-methyl-1-piperazinyl)-3-chinolincarbonsäure und deren pharmazeutisch verwendbare Säureadditionssalze, Alkalisalze, Erdalkalisalze oder Hydrate.

**Herstellungsbeispiele für die Ausgangsprodukte**

**Beispiel A**

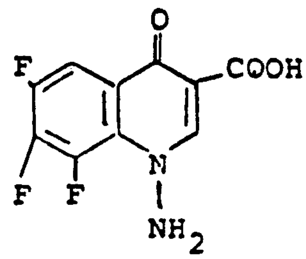

Eine Lösung von 16 g 2-(2,3,4,5-Tetrafluorbenzoyl)-3-ethoxyacrylsäurethylester in 70 ml Ethanol wird unter Kühlung mit Trockeneis/Aceton bei -25 bis -30°C zunächst tropfenweise mit 2,8 g Hydrazinhydrat und 30 Min. später mit 12,8 g Aceton versetzt. Man läßt langsam auf Raumtemperatur kommen und rührt dann noch 1 Stunde bei 20 bis 25°C. Das Lösungsmittel wird dann i. Vak. abdestilliert und der orangefarbene Rückstand aus Cyclohexan/Leichtbenzin umkristallisiert. Es werden 12,5 g 2-(2,3,4,5-Tetrafluorbenzoyl)-3-(2-propylidenhydrazino)acrylsäureethylester vom Schmelzpunkt 86°C erhalten.

10,38 g 2-(2,3,4,5-Tetrafluorbenzoyl)-3-(2-propylidenhydrazino)-acrylsäureethylester, 2 g Natriumfluorid und 60 ml Dimethylformamid werden 2,5 Stunden auf 150-160°C erhitzt. Das DMF wird i. Vak. abgezogen, der Rückstand in $CH_2Cl_2/H_2O$ aufgenommen und die $CH_2Cl_2$-Phase nach dem Trocknen mit $Na_2SO_4$ i. Vak. eingeengt. Die Umkristallisation aus Ethanol liefert 6 g 6,7,8-Trifluor-1 -(2-propyliden-amino)-1,4-dihydro-4-oxo-3-chinolincarbonsäureethylester vom Schmelzpunkt 166°C. Eine Suspension von 6 g Propylidenaminochinolincarbonsäureester in 72 ml Isopropanol und 0,7 g Wasser mit 0,16 g p-Toluolsulfonsäure 1 Stunde auf 70°C erhitzt. Dann werden bei Normaldruck etwa 50 ml abdestilliert und der Rückstand wird mit 25 ml Wasser versetzt. Man saugt kalt ab und wäscht mit einem Isopropanol/Wasser (1 : 1) Gemisch nach. Es werden 4,7 g 1-Amino-6,7,8-trifluor-1,4-dihydro-4-oxo-3-chinolincarbonsäureethylester vom Schmelzpunkt 217°C erhalten.

5,7 g 1-Amino-6,7,8-trifluor-1,4-dihydro-4-oxo-3-chinolincarbonsäureethylester werden mit 1,25 g Ätzkali und 100 ml Wasser 2,5 Stunden refluxiert. Man filtriert die warme Lösung und wäscht mit Wasser nach. Dann wird mit halbkonz. Salzsäure unter Eiskühlung auf pH = 1-2 angesäuert, der Niederschlag abgesaugt, mit Wasser gewaschen und im Vakuum bei 100°C getrocknet. Nach Umkristallisation aus Acetonitril erhält man 4 g 1-Amino-6,7,8-trifluor-1,4-dihydro-4-oxo-3-chinolincarbonsäure vom Schmelzpunkt 225-226°C.

**Herstellung des Ausgangsproduktes**

20,1 g Magnesiumspäne werden in 40 ml wasserfreiem Ethanol suspendiert. Man versetzt mit 4 ml Tetrachlorkohlenstoff und tropft, wenn die Reaktion in Gang gekommen ist, ein Gemisch von 132,2 g Malonsäurediethylester, 80 ml abs. Ethanol und 320 ml wasserfreiem Toluol bei 50 - 60°C zu. Nach dem Abklingen der Reaktion wird noch 2 h auf 60°C erhitzt, mit Trockeneis/Aceton auf -5 bis -10°C gekühlt und bei dieser Temperatur eine Lösung von 175,5 g 2,3,4,5-Tetrafluorbenzoylchlorid in 100 ml abs. Toluol langsam zugetropft. Man rührt 1 h bei 0 bis -5°C, läßt über Nacht auf Raumtemperatur kommen und läßt unter Eiskühlung ein Gemisch von 350 ml Eiswasser und 50 ml konz. Schwefelsäure zulaufen. Die Phasen werden getrennt und zweimal mit Toluol nachextrahiert. Die vereinigten Toluollösungen werden mit gesättigter Natriumchlorid-Lösung gewaschen, mit Natriumsulfat getrocknet und das Lösungsmittel im Vakuum abgezogen. Man erhält 284,8 g 2,3,4,5-Tetrafluorbenzoyl-malonsäurediethylester als Rohprodukt.

Eine Emulsion von 284,8 g rohem 2,3,4,5-Tetrafluorbenzoyl-malonsäurediethylester in 300 ml Wasser wird mit 0,3 g p-Toluolsulfonsäure versetzt. Man erhitzt unter gutem Rühren 4.5 h zum Sieden, extrahiert die erkaltete Emulsion mehrmals mit Methylenchlorid, wäscht die vereinigten Methylenchlorid-Lösungen einmal mit gesättigter Natriumchlorid-Lösung, trocknet mit Natriumsulfat und destilliert das Lösungsmittel im Vakuum ab. Die Fraktionierung des Rückstandes im Feinvakuum liefert 160,2 g 2,3,4,5-Tetrafluorbenzoyl-essigsäureethylester vom Siedepunkt 75-96°C/0,06-0,09 mbar.

Ein Gemisch von 110,75 g 2,3,4,5-Tetrafluorbenzoyl-essigsäureethylester, 93,5 g o-Ameisensäureethylester und 107 g Acetanhydrid wird 2 h auf 150°C erhitzt. Dann werden im Wasserstrahlvakuum und zuletzt im Feinvakuum bei einer Badtemperatur von 120°C die flüchtigen Bestandteile abdestilliert. Zurück bleiben 123,9 g roher 2,3,4,5-Tetrafluorbenzoyl)-3-ethoxy-acrylsäureethylester. Er ist genügend rein für die weiteren Umsetzungen.

**Beispiel B**

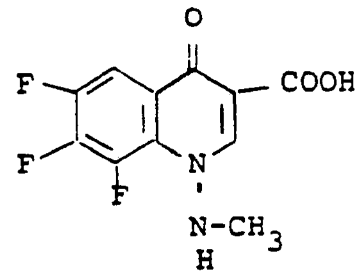

Eine Lösung von 16 g 2-(2,3,4,5-Tetrafluorbenzoyl)-3-ethoxy-acrylsäureethylester in 60 ml Ethanol wird unter Eiskühlung und Rühren tropfenweise mit 3,8 g 1-Formyl-1-methyl-hydrazin versetzt. Man rührt 1 Stunde bei Raumtemperatur, versetzt mit 60 ml Wasser, kühlt mit Eis, saugt den Niederschlag ab und wäscht ihn mit Wasser/Ethanol (1 : 1). Es werden 12 g 2-(2,3,4,5-Tetrafluorbenzoyl)-3-(2-formyl-2-methyl-hydrazino)-acrylsäureethylester vom Schmelzpunkt 92°C erhalten.

12 g der voranstehenden Verbindung werden mit 2,2 g Natriumfluorid und 100 ml DMF 2 Stunden auf 160°C erhitzt. Man gießt auf 400 ml Eiswasser, saugt ab, wäscht mit $H_2O$ nach und trocknet bei 100°C i. Vak.. Es werden 9,8 g 6,7,8-Trifluor-1-(formyl-methylamino)-1,4-dihydro-4-oxo-3-chinolin-carbonsäureethylester vom Schmelzpunkt 185°C erhalten.

Ein Gemisch von 9,6 g Formyl-methyl-amino-chinolincarbonsäureethylester, 150 ml Ethanol, 0,8 ml $H_2O$ und 4,1 ml Triethylamin wird 2 Stunden unter Rückfluß zum Sieden erhitzt. Dann werden 150 ml Eiswasser zugegeben, der Niederschlag wird abgesaugt und mit Ethanol/$H_2O$ (1 : 1) gewaschen. Es werden 7,5 g 6,7,8-Trifluor-1-methylamino-1,4-dihydro-4-oxo-3-chinolincarbonsäureethylester vom Schmelzpunkt 189°C erhalten.

7,5 g 1-Methylamino-3-chinolincarbonsäureester, 55 ml Eisessig, 40 ml Wasser und 6 ml konz. Schwefelsäure werden 2 Stunden refluxiert. Die erkaltete Lösung wird auf Eis gegossen, der Niederschlag abgesaugt, mit Wasser gewaschen und i. Vak. bei 100°C getrocknet. Nach Umkristallisation aus DMF/Ethanol werden 4,8 g 6,7,8-Trifluor-1-methylamino-1,4-dihydro-4-oxo-3-chinolincarbonsäure vom Schmelzpunkt 240°C erhalten.

**Beispiel C**

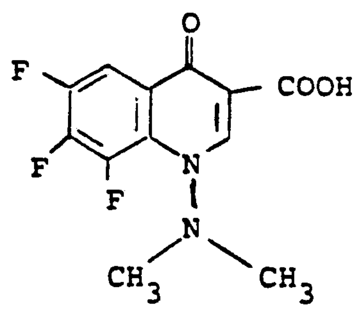

Eine Lösung von 16 g 2-(2,3,4,5-Tetrafluorbenzoyl)-3-ethoxyacrylsäureethylester in 60 ml Ethanol wird unter Eiskühlung und Rühren tropfenweise mit 3,1 g 1,1-Dimethylhydrazin versetzt. Man rührt 1 Stunde bei Raumtemperatur, versetzt mit 60 ml $H_2O$, kühlt mit Eis, saugt den Niederschlag ab und wäscht ihn mit Wasser/Ethanol (1 : 1). Es werden 12,9 g 2-(2,3,4,5-Tetrafluorbenzoyl)-3-(2,2-dimethylhydrazino)-acrylsäureethylester vom Schmelzpunkt 81°C erhalten.

12,9 g der voranstehenden Verbindung werden mit 2,5 g Natriumfluorid und 100 ml DMF 2 Stunden auf 160°C erhitzt. Man gießt auf 400 ml Eiswasser, saugt ab, wäscht mit Wasser nach und trocknet bei 100°C i. Vak. Es werden 11,4 g 6,7,8-Trifluor-1-dimethylamino-1,4-dihydro-4-oxo-3-chinolincarbonsäureethylester vom Schmelzpunkt 172°C erhalten.

11,2 g 6,7,8-Trifluor-1-dimethylamino-1,4-dihydro-4-oxo-3-chinolincarbonsäureester, 70 ml Eisessig, 55 ml Wasser und 7,8 ml konz. Schwefelsäure werden 2 Stunden refluxiert. Die erkaltete Lösung wird auf Eis gegossen, der Niederschlag abgesaugt, mit Wasser gewaschen und i. Vak. bei 100°C getrocknet. Nach Umkristallisation aus DMF/Ethanol werden 8,5 g 6,7,8-Trifluor-1-dimethylamino-1,4-dihydro-4-oxo-3-chinolincarbonsäure vom Zersetzungspunkt 293-295°C erhalten.

Analog Beispielen A-C können hergestellt werden:

**Beispiel D:** 1-Methylamino-7-chlor-6-fluor-8-nitro-1,4-dihydro-4-oxo-3-chinolincarbonsäure vom Zersetzungspunkt 270-272° C

**Herstellungsbeispiele für die erfindungsgemäßen Endprodukte:**

**Beispiel 1**

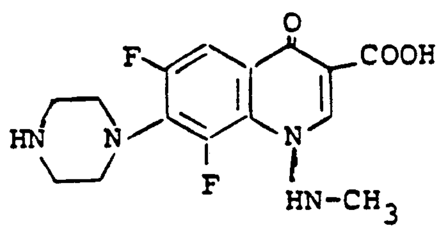

Ein Gemisch aus 1,95 g 1-Methylamino-6,7,8-trifluor-1,4-dihydro-4-oxo-3-chinolincarbonsäure, 3,05 g wasserfreiem Piperazin und 20 ml trockenem Pyridin wird 6 Stunden refluxiert. Das Lösungsmittel wird i. Vak. abgezogen, der Rückstand in 20 ml Wasser aufgenommen, unter Eiskühlung mit halbkonz. Salzsäure auf pH = 7-8 gestellt, der Niederschlag abgesaugt, mit Eiswasser gewaschen und bei 100° C im Vakuum getrocknet. Man erhält 1,8 g 1-Methylamino-6,8-difluor-7-(1-piperazinyl)-1,4-dihydro-4-oxo-3-chinolincarbonsäure vom Zersetzungspunkt 276-278° C.

Analog Beispiel 1 werden folgende Verbindungen erhalten:

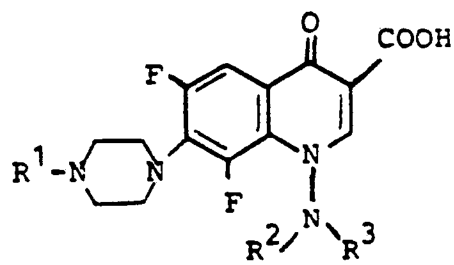

| Beispiel | $R^1$ | $R^2$ | $R^3$ | Zersetzungspunkt (° C) |
|---|---|---|---|---|
| 2 | $CH_3$ | $CH_3$ | $CH_3$ | 246 |
| 3 | $CH_3$ | H | $CH_3$ | 215 |
| 4 | H | H | H | 286 |

**Beispiel 5**

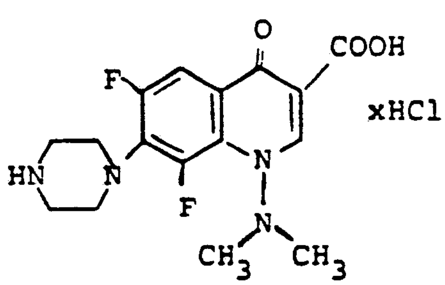

Ein Gemisch von 2,86 g 1-Dimethylamino-6,7,8-trifluor-1,4-dihydro-4-oxo-3-chinolincarbonsäure, 4,3 g wasserfreiem Piperazin und 30 ml trockenem Pyridin wird 6 Stunden unter Rückfluß zum Sieden erhitzt. Das Pyridin wird dann im Vakuum abgezogen und der Rückstand mit 10 ml Wasser versetzt. Der pH-Wert der Lösung wird mit konz. Salzsäure auf ca. 1 gestellt, der Niederschlag kalt abgesaugt und mit wenig eiskalter 10-proz. Salzsäure und Ethanol gewaschen. Nach dem Trocknen im Vakuum bei 100°C werden 1,5 g 1-Dimethylamino-6,8-difluor-7-(1-piperazinyl)-1,4-dihydro-4-oxo-3-chinolincarbonsäure-hydrochlorid vom Zersetzungspunkt 284-286°C erhalten.

**Beispiel 6**

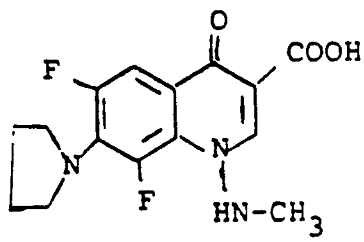

Eine Mischung von 2,72 g 1-Methylamino-6,7,8-trifluor-1,4-dihydro-4-oxo-3-chinolincarbonsäure, 2,3 g Pyrrolidin und 30 ml Pyridin wird 6 Stunden zum Sieden erhitzt. Das Lösungsmittel wird im Vakuum abdestilliert und der Rückstand mit 20 ml Wasser versetzt. Unter Eiskühlung wird mit 10-proz. Salzsäure der pH-Wert auf 1-2 eingestellt, der Niederschlag abgesaugt, mit Wasser gewaschen und i. Vak. bei 100°C getrocknet. Nach der Umkristallisation aus Glykolmonomethylether/Ethanol werden 2,4 g 1-Methylamino-6,8-difluor-7-(1-pyrrolidinyl)-1,4-dihydro-4-oxo-3-chinolincarbonsäure vom Zersetzungspunkt 282-283°C erhalten.

Analog Beispiel 6 werden die folgenden Verbindungen erhalten:

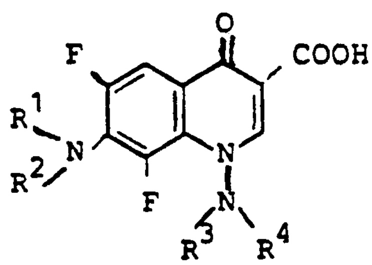

| Beispiel | $R^1$ $R^2$ | $R^3$ | $R^4$ | Zersetzungspunkt (0°C) |
|---|---|---|---|---|
| 7 | $-CH_2CH_2CH_2CH_2-$ | H | H | 274 |
| 8 | $-CH_2CH_2CH_2CH_2-$ | $CH_3$ | $CH_3$ | 279 |
| 9 | $-CH_2CH_2OCH_2CH_2-$ | $CH_3$ | $CH_3$ | 284 |
| 10 | $-CH_2CH_2OCH_2CH_2-$ | H | H | 262 |

**Beispiel 11**

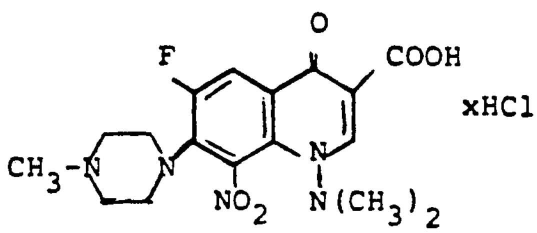

Ein Gemisch von 3,3 g 1-Dimethylamino-7-chlor-6-fluor-8-nitro-1,4-dihydro-4-oxo-3-chinolincarbonsäure, 3,1 g 1-Methylpiperazin und 25 ml Dioxan wird 3 Stunden refluxiert. Das Lösungsmittel wird im Vakuum abgezogen, der Rückstand in 25 ml $H_2O$ aufgeschlämmt und mit 10-proz. Salzsäure auf pH = 1 gestellt. Der Niederschlag wird kalt abgesaugt und mit wenig kalter 10-proz. Salzsäure sowie Ethanol gewaschen. Es werden 3,6 g 1-Dimethylamino-6-fluor-8-nitro-7-(4-methyl-1-piperazinyl)-1,4-dihydro-4-oxo-3-chinolincarbonsäure-hydrochlorid vom Zersetzungspunkt 292-294° C erhalten.

Analog Beispiel 11 werden folgende Verbindungen erhalten:

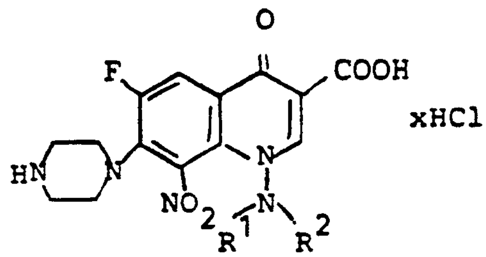

| Beispiel | $R^1$ | $R^2$ | Zersetzungspunkt (° C) |
|---|---|---|---|
| 12 | H | $CH_3$ | 284 |
| 13 | $CH_3$ | $CH_3$ | 280 |

**Beispiel 14**

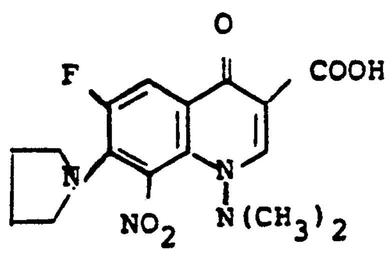

Man setzt 3,3 g 1-Dimethylamino-7-chlor-6-fluor-8-nitro-1,4-dihydro-4-oxo-3-chinolincarbonsäure mit 6 g Pyrrolidin analog Beispiel 11 um und isoliert nach dem Ansäuern mit 10-proz. Salzsäure auf pH = 1 3,4 g 1-Dimethylamino-6-fluor-8-nitro-7(1-pyrrolidinyl)-1,4-dihydor-4-oxo-3-chinolincarbonsäure. Nach Um kristallisation aus Glykolmonomethylether schmelzen die Kristalle bei 266-268° C unter Zersetzung.

**Beispiel 15**

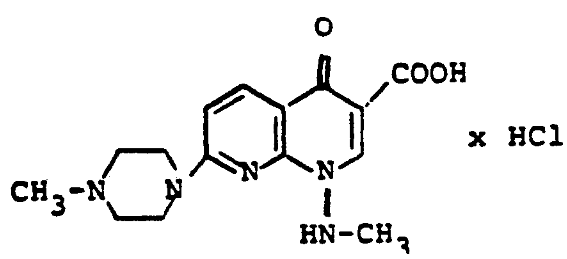

Eine Suspension von 2,53 g 1-Methylamino-7-chlor-1,4-dihydro-4-oxo-1,8-naphthyridin-3-carbonsäure in 30 ml Ethanol wird unter Rühren tropfenweise mit 3 g 1-Methylpiperazin versetzt, wobei die Säure bei leicht exothermer Reaktion in Lösung geht. Man erhitzt 1 Stunde zum Sieden, destilliert das Ethanol ab und löst das Reaktionsprodukt in 1 N Natronlauge. Die filtrierte Lösung wird mit 10-proz. Salzsäure auf pH = 6 eingestellt. Der Niederschlag wird kalt abgesaugt, mit wenig kaltem Wasser und Ethanol gewaschen und im Vakuum bei 80°C getrocknet. Es werden 3,1 g 1-Methylamino-7-(4-methyl-1-piperazinyl)-1,4-dihydro-4-oxo-1,8-naphthyridin-3-carbonsäure-hydrochlorid vom Zersetzungspunkt 328°C erhalten.

Analog Beispiel 15 werden folgende Verbindungen erhalten:

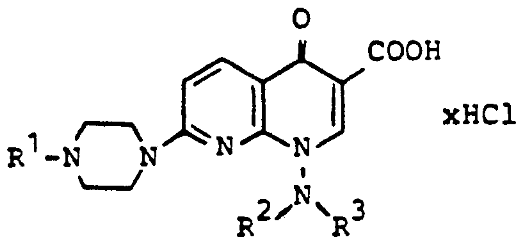

| Beispiel | $R^1$ | $R^2$ | $R^3$ | Zersetzungspunkt (°C) |
|---|---|---|---|---|
| 16 | H | H | $CH_3$ | 316 |
| 17 | $HOCH_2CH_2$- | H | $CH_3$ | 313 |
| 18 | $CH_3$ | $CH_3$ | $CH_3$ | 276 (Acetat) |

**Beispiel 19**

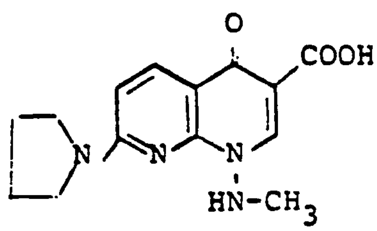

2,6 g 1-Methylamino-7-chlor-1,4-dihydro-4-oxo-1,8-naphthyridin-3-carbonsäure werden in 60 ml Ethanol suspendiert. Unter Eiskühlung und Rühren versetzt man tropfenweise mit 5 g Pyrrolidin. Dann wird 10 Minuten bei Raumtemperatur und 1 Stunde bei Rückfluß gerührt. Das Ethanol wird i. Vak. abgezogen, der Rückstand in wenig 1N Natronlauge gelöst, filtriert und mit 10-proz. Salzsäure angesäuert. Der Niederschlag wird abgesaugt, mit Wasser gewaschen und bei 80°C i. Vak. getrocknet. Nach Umkristallisation aus DMF werden 2,2 g 1-Methylamino-7-(1-pyrrolidinyl)-1,4-dihydro-4-oxo-1,8-naphtyridin-3-carbonsäure vom Zersetzungspunkt 338-340°C erhalten.

Analog Beispiel 19 werden folgende Verbindungen erhalten:

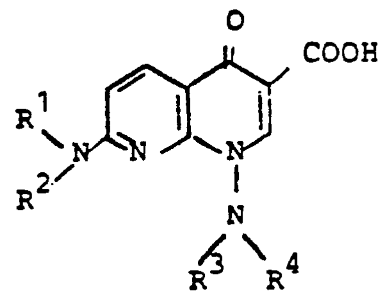

| Beispiel | $R^1$ | $R^2$ | $R^3$ | $R^4$ | Zersetzungspunkt (0° C) |
|---|---|---|---|---|---|
| 20 | $-(CH_2)_5-$ | | H | $CH_3$ | 278 |
| 21 | $-(CH_2)_2O(CH_2)_2-$ | | H | $CH_3$ | 288 |

Die erfindungsgemäßen Verbindungen zeigen ein breites antibakterielles Spektrum gegen gram-positive und gram-negative Keime, insbesondere gegen Enterobakteriaceen, vor allem auch gegen solche, die resistent sind gegen verschiedene Antibiotika wie z. B. Penicilline, Cephalosporine, Aminoglykoside, Sulfonamide, Tetracycline.

Die erfindungsgemäßen Verbindungen weisen bei geringer Toxizität eine starke und breite antimikrobielle Wirksamkeit auf. Diese Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z. B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Micrococcaceae, wie Staphylokokken, z. B. Staphylococcus aureus, Staph. epidermidis, (Staph. = Staphylococcus), Lactobacteriaceae, wie Streptokokken, z. B. Streptococcus pyogenes, α- bzw. β-hämolysierende Streptokokken, nicht (γ-)-hämolysierende Streptokokken, Enterokokken und Diplococcus pneumoniae (Pneumokokken) (Str. = Streptococcus); Enterobacteriaceae, wie Escherichiae-Bakterien der Coli-Gruppe: Escherichia-Bakterien, z. B. Escherichia coli, Enterobacter-Bakterien, z. B. E.aerogenes, E.cloacae, Klebsiella-Bakterien, z. B. K-pneumoniae, Serratia, z. B. Serratia marcescens (E. = Enterobacter) (K. = Klebsiella), Proteae-Bakterien der Proteus-Gruppe: Proteus, z. B. Proteus vulgaris, Pr. morganii, Fr. rettgeri, Fr. mirabilis, (Pr. = Proteus):

Pseudomonadaceae, wie Pseudomonas-Bakterien, z. B. Pseudomonas aeruginosa, (PS. = Pseudomonas);

Bacteroidaceae, wie Bacteroides-Bakterien, z. B. Bacteroides fragilis, (B. = Bacteroides).

Mykoplasmen, z. B. Mykoplasme pneumoniae.

Die obige Aufzählung von Erregern ist lediglich beispielhaft und keineswegs beschränkend aufzufassen.

Als Krankheiten, die durch die erfindungsgemäßen Verbindungen verhindert, gebessert und/oder geheilt werden können seien beispielsweise genannt:

Erkrankungen der Atmungswege und des Rachenraumes;

Otitis; Pharyngitis; Pneumonie; Peritonitis; Pyelonephritis; Cystitis; Endocarditis; Systeminfektionen; Bronchitis; Arthritis; lokale Infektionen, septische Erkrankungen.

Zur Erfindung gehören pharmazeutische Zubereitungen, die neben nichttoxischen, inerten pharmazeutisch geeigneten Trägerstoffen einen oder mehrere erfindungsgemäße Verbindungen enthalten oder die aus einem oder mehreren erfindungsgemäßen Wirkstoffen bestehen sowie Verfahren zur Herstellung dieser Zubereitungen.

Zur Erfindung gehören auch pharmazeutische Zubereitungen in Dosierungseinheiten. Dies bedeutet, daß die Zubereitungen in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B Glycerin, (d) Sprengmittel, z. B. Agar-Agar, Calciumcarbonat und Natriumcarbonat, (e) Lösungsverzögerer, z. B. Paraffin und (f) Resorptionsbeschleuniger, z. B. quarternäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffen enthalten, z. B. Polyethylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyethylenglykole, Silcone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel z. B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Ethylalkohol, Propylenglykol, Suspendiermittel, z. B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z. B. Pfefferminzöl und Eukalyptusöl und Süßmittel, z. B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gewichtsprozent der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise oral oder parenteral wie intravenös oder intramuskulär appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe, vorzugsweise in Mengen von etwa 1 bis etwa 250, insbesondere 3 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch

eine Förderung des Wachstums und einer Verbesserung der Verwendung des Futters erreicht werden.

Zwar sind aus der Europäischen Patentanmeldung 0 090 424 bereits 1,7-Diamino-6-fluor-chinolon-3-carbonsäuren bekannt. Die erfindungsgemäßen neuen 1,7-Diamino-1,4-dihydro-4-oxo-3-(aza)chinolincarbonsäuren sind diesen bekannten Verbindungen jedoch bezüglich ihrer antibakteriellen Wirkung und hinsichtlich der erreichbaren Serumspiegel überlegen, wie aus den nachstehenden Tabellen 1, 2 und 3 hervorgeht.

Tabelle 1 zeigt MHK-Werte der erfindungsgemäßen Verbindung des Beispiels 3 (I) im Vergleich zu dem analogen aus EP-A-0 090 424 bekannten 6-Monofluorderivat (II). Die Werte liegen in den meisten Fällen gleich, bei einigen Stämmen etwa 2-4fach besser. In Untersuchungen zur Pharmakokinetik bei der Maus nach oraler und parenteraler Gabe (siehe Tabelle 2 + 3) konnte gezeigt werden, daß BAY Q 8805 höhere Serumspiegel liefert als das Konkurrenzprodukt. Damit ist bei etwa gleicher in vitro Wirksamkeit in vivo durch die bessere Bioverfügbarkeit nach oraler und parenteraler Gabe eine höhere therapeutische Wirkwahrscheinlichkeit gegeben.

**Tabelle 1**

| Stamm | MHK mcg/ml II | I |
|---|---|---|
| E. coli 455/7 | 8 | 8 |
| E. coli A 261 | 0,06 | 0,06 |
| Klebsiella 6179 | 0,25 | 0,125 |
| Klebsiella 57 USA | 0,25 | 0,125 |
| Proteus mir. 8223 | 4 | 4 |
| Proteus mir. 8175 | 0,125 | 0,25 |
| Providencia 12052 | 16 | 4 |
| Pseudomonas W. | 1 | 1 |

Agarverdünnungstest / Isosensitestmedium

**Tabelle 2**

Serumspiegel Maus nach po - Gabe von 5 mg/kg

| | mcg/ml Serum | | | |
|---|---|---|---|---|
| Präparat | 15' | 30' | 1h | 2h |
| I | 0,9 | 1,2 | 0,6 | 0,25 |
| II | 0,7 | 0,58 | 0,32 | 0,1 |

**Tabelle 3**

Serumspiegel Maus nach <u>subkutaner</u> Gabe von 5 mg/kg

| | mcg/ml Serum | | | |
|---|---|---|---|---|
| Präparat | 15' | 30' | 1h | 2h |
| I | 1,2 | 1,4 | 0,92 | 0,3 |
| II | 0,84 | 1,3 | 0,45 | 0,1 |

**Patentansprüche**

1. 1,7-Diamino-1,4-dihydro-4-oxo-3-(aza)chinolincarbonsäuren der allgemeinen Formel I

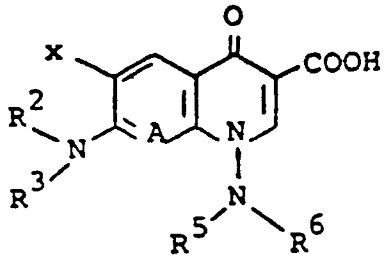

(I)

in der
A entweder (Stickstoff oder C-R[1] bedeutet, wobei $R^1$ für die Nitrogruppe oder Halogen steht,
$R^2$ und $R^3$ gleich oder verschieden sein können und für einen $C_1$-$C_3$-Alkylrest stehen und weiterhin gemeinsam mit dem Stickstoffatom, an das sie gebunden sind, einen 5- oder 6-gliedrigen heterocyclischen Ring bilden können, der als Ringglied zusätzlich die Atome oder Gruppen -O-, -S-, -SO-, $-SO_2-$ oder = N-$R^4$ enthalten kann und der gegebenenfalls an den Kohlenstoffatomen ein- bis dreifach durch $C_1$-$C_3$-Alkyl, Hydroxy, Alkoxy mit 1-3 Kohlenstoffatomen, Amino, Methylamino oder Ethylamino substituiert sein kann, wobei jeweils ein Kohlenstoffatom nur einen Substituenten tragen kann,
wobei
$R^4$ für Wasserstoff, eine verzweigte oder unverzweigte Alkyl- oder Alkenylgruppe mit 1 bis 4 Kohlenstoffatomen, die gegebenenfalls durch eine Hydroxy-, Alkoxy-, Alkylmerkapto-, Alkylamino- oder Dialkylamino-gruppe mit 1 bis 3 Kohlenstoffatomen für einen Alkylrest, die Cyangruppe, die Alkoxycarbonylgruppe mit 1 bis 4 Kohlenstoffatomen im Alkoholteil substituiert sein kann, die Benzyloxycarbonylgruppe, eine gegebenenfalls im Phenylrest substituierte Phenylalkylgruppe mit bis zu 4 Kohlenstoffatomen im aliphatischen Teil, einen gegebenenfalls durch Hydroxy, Methoxy, Chlor und Fluor ein- oder zweifach substituierten Phenylrest, einen Oxoalkylrest mit bis zu 6 Kohlenstoffatomen sowie einen Cycloalkylalkylrest mit bis zu 6 Kohlenstoffatomen im cyclischen Teil und bis zu 3 Kohlenstoffatomen im cyclischen Teil steht, sowie $R^5$ und $R^6$ gleich oder verschieden sein können und Wasserstoff oder eine Alkylgruppe mit 1-4 Kohlenstoffatomen bedeuten und ferner X für Wasserstoff, die Nitrogruppe oder Halogen steht,
und deren pharmazeutisch verwendbare Salze.

2. 1,7-Diamino-1,4-dihydro-4-oxo-3-(aza)chinolincarbonsäuren der allgemeinen Formel I in Anspruch 1, in denen X und $R^1$ Fluor oder Chlor bedeuten und $R^2$, $R^3$, $R^4$, $R^5$ und $R^6$ die oben angegebene Bedeutung besitzen.

3. 1,7-Diamino-1,4-dihydro-4-oxo-3-(aza)chinolincarbonsäuren der allgemeinen Formel I in Anspruch 1, in denen A entweder Stickstoff oder C-$R^1$ bedeutet, wobei $R^1$ für die Nitrogruppe steht und X, $R^2$, $R^3$, $R^4$ , $R^5$ und $R^6$ die oben angegebene Bedeutung besitzen.

4. 1-Methylamino-6,8-difluor-7-(4-methylpiperazinyl)-1,4-dihydro-4-oxo-3-chinolincarbonsäure.

5. 1-Methylamino-6,8-difluor-7-(1-piperazinyl)-1,4-dihydro-4-oxo-3-chinolincarbonsäure.

6. 1-Amino-6,8-difluor-7-(1-piperazinyl)-1,4-dihydro -4-oxo-3-chinolincarbonsäure.

7. 1-Methylamino-6,8-difluor-7-(1-pyrrolidinyl)-1,4-dihydro-4-oxo-3-chinolincarbonsäure.

8. 1,7-Diamino-1,4-dihydro-4-oxo-3-(aza)chinolincarbonsäuren der allgemeinen Formel I

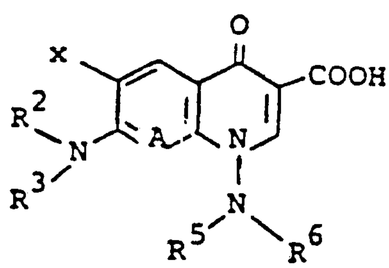

(I)

in der
A entweder Stickstoff oder C-$R^1$ bedeutet, wobei $R^1$ für die Nitrogruppe oder Halogen steht,
$R^2$ und $R^3$ gleich oder verschieden sein können und für einen $C_1C_3$-Alkylrest stehen und weiterhin gemeinsam mit dem Stickstoffatom, an das sie gebunden sind, einen 5- oder 6-gliedrigen heterocyclischen Ring bilden können, der als Ringglied zusätzlich die Atome oder Gruppen -O-, -S-, -SO-, $-SO_2-$ oder = N-$R^4$ enthalten kann und der gegebenenfalls an den Kohlenstoffatomen ein- bis dreifach durch $C_1$-$C_3$-Alkyl, Hydroxy, Alkoxy mit 1-3 Kohlenstoffatomen, Amino, Methylamino oder Ethylamino substituiert sein kann, wobei jeweils ein Kohlenstoffatom nur einen Substituenten tragen kann,
wobei
$R^4$ für Wasserstoff, eine verzweigte oder unverzweigte Alkyl- oder Alkenylgruppe mit 1 bis 4 Kohlenstoffatomen, die gegebenenfalls durch eine Hydroxy-, Alkoxy-, Alkylmerkapto-, Alkylamino- oder Dialkylamino-gruppe mit 1 bis 3 Kohlenstoffatomen für einen Alkylrest, die Cyangruppe, die Alkoxycarbonylgruppe mit 1 bis 4 Kohlenstoffatomen im Alkoholteil substituiert sein kann, die Benzyloxycarbonylgruppe, eine gegebenenfalls im Phenylrest substituierte Phenylalkylgruppe mit bis zu 4

Kohlenstoffatomen im aliphatischen Teil, einen gegebenenfalls durch Hydroxy, Methoxy, Chlor und Fluor ein- oder zweifach substituierten Phenylrest, einen Oxoalkylrest mit bis zu 6 Kohlenstoffatomen sowie einen Cycloalkylalkylrest mit bis zu 6 Kohlenstoffatomen im cyclischen Teil und bis zu 3 Kohlenstoffatomen im acyclischen Teil steht, sowie $R^5$ und $R^6$ gleich oder verschieden sein können und Wasserstoff oder eine Alkylgruppe mit 1-4 Kohlenstoffatomen bedeuten und ferner X für Wasserstoff, die Nitrogruppe oder Halogen steht,

und deren pharmazeutisch verwendbare Salze zur Verwendung bei der Bekämpfung bakterieller Erkrankungen.

9. Verfahren zur Herstellung von 1,7-Diamino-1,4-dihydro -4-oxo-3-(aza)chinolincarbonsäuren der allgemeinen Formel I

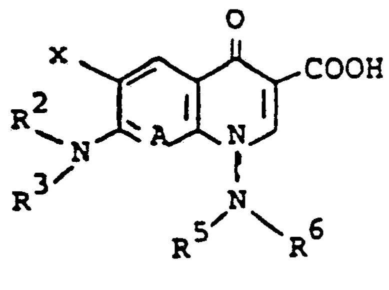

(I)

in der

A entweder Stickstoff oder C-$R^1$ bedeutet, wobei $R^1$ für die Nitrogruppe oder Halogen steht,

$R^2$ und $R^3$ gleich oder verschieden sein können und für einen $C_1$-$C_3$-Alkylrest stehen und weiterhin gemeinsam mit dem Stickstoffatom, an das sie gebunden sind, einen 5- oder 6-gliedrigen heterocyclischen Ring bilden können, der als Ringglied zusätzlich die Atome oder Gruppen -O-, -S-, -SO-, -$SO_2$- oder =N-$R^4$ enthalten kann und der gegebenenfalls an den Kohlenstoffatomen ein- bis dreifach durch $C_1$-$C_3$-Alkyl, Hydroxy, Alkoxy mit 1-3 Kohlenstoffatomen, Amino, Methylamino oder Ethylamino substituiert sein kann, wobei jeweils ein Kohlenstoffatom nur einen Substituenten tragen kann, wobei

$R^4$ für Wasserstoff, eine verzweigte oder unverzweigte Alkyl- oder Alkenylgruppe mit 1 bis 4 Kohlenstoffatomen, die gegebenenfalls durch eine Hydroxy-, Alkoxy-, Alkylmerkapto-, Alkylamino- oder Dialkylamino-gruppe mit 1 bis 3 Kohlenstoffatomen für einen Alkylrest, die Cyangruppe, die Alkoxycarbonylgruppe mit 1 bis 4 Kohlenstoffatomen im Alkoholteil substituiert sein kann, die Benzyloxycarbonylgruppe, eine gegebenenfalls im Phenylrest substituierte Phenylalkylgruppe mit bis zu 4 Kohlenstoffatomen im aliphatischen Teil, einen gegebenenfalls durch Hydroxy, Methoxy, Chlor und Fluor ein- oder zweifach substituierten Phenylrest, einen Oxoalkylrest mit bis zu 6 Kohlenstoffatomen sowie einen Cycloalkylalkylrest mit bis zu 6 Kohlenstoffatomen im cyclischen Teil und bis zu 3 Kohlenstoffatomen im acyclischen Teil steht, sowie $R^5$ und $R^6$ gleich oder verschieden sein können und Wasserstoff oder eine Alkylgruppe mit 1-4 Kohlenstoffatomen bedeuten und ferner X für Wasserstoff, die Nitrogruppe oder Halogen steht,

und deren pharmazeutisch verwendbare Salzen,

dadurch gekennzechnet, daß man 1-Amino-chinoloncarbonsäuren der allgemeinen Formel II

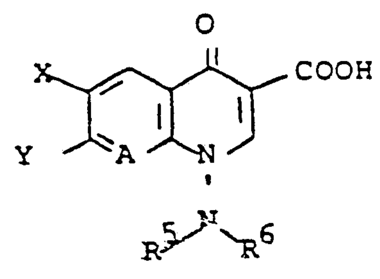

(II)

in der

X, A und $R^5$, $R^6$ die oben angegebene Bedeutung haben und Y für Halogen steht, mit Aminen der allgemeinen Formel III

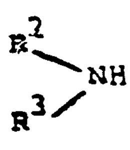

(III)

in welcher

$R^2$ und $R^3$ die oben angegebene Bedeutung haben, gegebenenfalls in Gegenwart von Säurebindern, umsetzt.

10. Verwendung von 1,7-Diamino-1,4-dihydro-4-oxo-3-(aza)chinolincarbonsäuren der allgemeinen Formel I

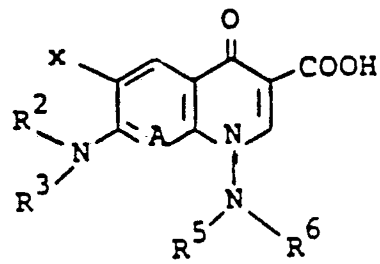

(I)

in der

A entweder Stickstoff oder C-$R^1$ bedeutet, wobei $R^1$ für die Nitrogruppe oder Halogen steht,

$R^2$ und $R^3$ gleich oder verschieden sein können und für einen $C_1$-$C_3$-Alkylrest stehen und weiterhin gemeinsam mit dem Stickstoffatom, an das sie gebunden sind, einen 5- oder 6-gliedrigen heterocyclischen Ring bilden können, der als Ringglied zusätzlich die Atome oder Gruppen -O-, -S-, -SO-, -$SO_2$- oder = N-$H^4$ enthalten kann und der gegebenenfalls an den Kohlenstoffatomen ein- bis dreifach durch $C_1$-$C_3$-Alkyl, Hydroxy, Alkoxy mit 1-3 Kohlenstoffatomen, Amino, Methylamino oder Ethylamino substituiert sein kann, wobei jeweils ein Kohlenstoffatom nur einen Substituenten tragen kann, wobei

$R^4$ für Wasserstoff, eine verzweigte oder unverzweigte Alkyl- oder Alkenylgruppe mit 1 bis 4 Kohlenstoffatomen, die gegebenenfalls durch eine Hydroxy-, Alkoxy-, Alkylmerkapto-, Alkylamino- oder Dialkylamino-gruppe mit 1 bis 3 Kohlenstoffatomen für einen Alkylrest, die Cyangruppe, die Alkoxycarbonylgruppe mit 1 bis 4 Kohlenstoffatomen im Alkoholteil substituiert sein kann, die Benzyloxycarbonylgruppe, eine gegebenenfalls im Phenylrest substituierte Phenylalkylgruppe mit bis zu 4 Kohlenstoffatomen im aliphatischen Teil, einen gegebenenfalls durch Hydroxy, Methoxy, Chlor und Fluor ein- oder zweifach substituierten Phenylrest, einen Oxoalkylrest mit bis zu 6 Kohlenstoffatomen sowie einen Cycloalkylalkylrest mit bis zu 6 Kohlenstoffatomen im cyclischen Teil und bis zu 3 Kohlenstoffatomen im acyclischen Teil steht, sowie $R^5$ und $R^6$ gleich oder verschieden sein können und Wasserstoff oder eine Alkylgruppe mit 1-4 Kohlenstoffatomen bedeuten und ferner X für Wasserstoff, die Nitrogruppe oder Halogen steht,

und deren pharmazeutisch verwendbare Salzen zur Herstellung von Arzneimitteln.

## Claims

1. 1,7-Diamino-1,4-dihydro-4-oxo-3-(aza)quinolinecarboxylic acids of the general formula I

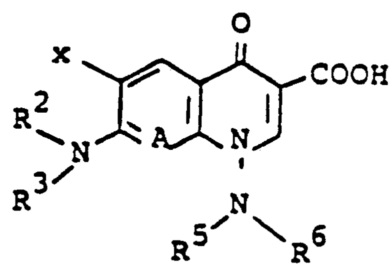

(I)

in which

A denotes either nitrogen or C-$R^1$,

wherein

$R^1$ represents the nitro group or halogen,

$R^2$ and $R^3$ can be identical or different and represent a $C_1$-$C_3$-alkyl radical, or furthermore, together with the nitrogen atom to which they are bonded, can form a 5-membered or 6-membered heterocyclic ring, which can additionally contain the atoms or groups -O-, -S-, -SO-, -$SO_2$- or = N-$R^4$ as a ring member and can optionally be mono-, di- or tri-substituted on the carbon atoms by $C_1$-$C_3$-alkyl, hydroxyl, alkoxy with 1-3 carbon atoms, amino, methylamino or ethylamino, it being possible for a carbon atom in each case to carry only one substituent,

wherein

$R^4$ represents hydrogen, a branched or straight-chain alkyl or alkenyl group which has 1 to 4 carbon atoms and can optionally be substituted by a hydroxyl, alkoxy, alkylmercapto, alkylamino or dialkylamino group with 1 to 3 carbon atoms for an alkyl radical, the cyano group or the alkoxycarbonyl group with 1 to 4 carbon atoms in the alcohol part, or the benzyloxycarbonyl group, a phenylalkyl group which has up to 4 carbon atoms in the aliphatic part and is optionally substituted in the phenyl radical, a phenyl radical which is optionally

monosubstituted or disubstituted by hydroxyl, methoxy, chlorine and fluorine, an oxoalkyl radical with up to 6 carbon atoms or a cyclo-alkyl-alkyl radical which has up to 6 carbon atoms in the cyclic part and up to 3 carbon atoms in the acyclic part, $R^5$ and $R^6$ can be identical or different and denote hydrogen or an alkyl group with 1 to 4 carbon atoms, and furthermore X represents hydrogen, the nitro group or halogen, and pharmaceutically useful salts thereof.

2. 1,7-Diamino-1,4-dihydro-4-oxo-3-(aza)quinolinecarboxylic acids of the general formula I in Claim 1, in which
X and $R^1$ denote fluorine or chlorine, and
$R^2$, $R^3$, $R^4$, $R^5$ and $R^6$ have the abovementioned meaning.

3. 1,7-Diamino-1,4-dihydro-4-oxo-3-(aza)quinolinecarboxylic acids of the general formula I in Claim 1, in which
A denotes nitrogen or C-$R^1$,
wherein
$R^1$ represents the nitro group, and
X, $R^2$, $R^3$, $R^4$, $R^5$ and $R^6$ have the abovementioned meaning.

4. 1-Methylamino-6,8-difluoro-7-(4-methylpiperazinyl)-1,4-dihydro-4-oxo-3-quinolinecarboxylic acid.

5. 1-Methylamino-6,8-difluoro-7-(1-piperazinyl)-1,4-dihydro-4-oxo-3-quinolinecarboxylic acid.

6. 1-Amino-6,8-difluoro-7-(1-piperazinyl)-1,4-dihydro-4-oxo-3-quinolinecarboxylic acid.

7. 1-Methylamino-6,8-difluoro-7-(1-pyrrolidinyl)-1,4-dihydro-4-oxo-3-quinolinecarboxylic acid.

8. 1,7-Diamino-1,4-dihydro-4-oxo-3-(aza)quinolinecarboxylic acids of the general formula I

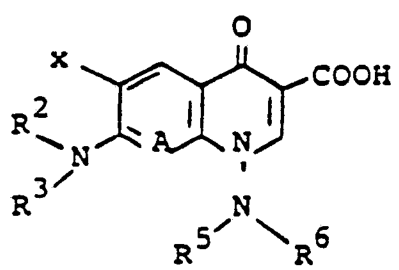

(I)

in which
A denotes either nitrogen or C-$R^1$,
wherein
$R^1$ represents the nitro group or halogen,
$R^2$ and $R^3$ can be identical or different and represent a $C_1$-$C_3$-alkyl radical, or furthermore, together with the nitrogen atom to which they are bonded, can form a 5-membered or 6-membered heterocyclic ring, which can additionally contain the atoms or groups -O-, -S-, -SO-, -$SO_2$- or = N-$R^4$ as a ring member and can optionally be mono-, di- or tri-substituted on the carbon atoms by $C_1$-$C_3$-alkyl, hydroxyl, alkoxy with 1-3 carbon atoms, amino, methylamino or ethylamino, it being possible for a carbon atom in each case to carry only one substituent,
wherein
$R^4$ represents hydrogen, a branched or straight-chain alkyl or alkenyl group which has 1 to 4 carbon atoms and can optionally be substituted by a hydroxyl, alkoxy, alkylmercapto, alkylamino or dialkylamino group with 1 to 3 carbon atoms for an alkyl radical, the cyano group or the alkoxycarbonyl group with 1 to 4 carbon atoms in the alcohol part, or the benzyloxycarbonyl group, a phenylalkyl group which has up to 4 carbon atoms in the aliphatic part and is optionally substituted in the phenyl radical, a phenyl radical which is optionally monosubstituted or disubstituted by hydroxyl, methoxy, chlorine and fluorine, an oxoalkyl radical with up to 6 carbon atoms or a cycloalkylalkyl radical which has up to 6 carbon atoms in the cyclic part and up to 3 carbon atoms in the acyclic part, $R^5$ and $R^6$ can be identical or different and denote hydrogen or an alkyl group with 1 to 4 carbon atoms, and furthermore X represents hydrogen, the nitro group or halogen, and pharmaceutically useful salts thereof, for use in combating bacterial diseases.

9. Process for the preparation of 1,7-diamino-1,4-dihydro-4-oxo-3-(aza)quinolinecarboxylic acids of the general formula I

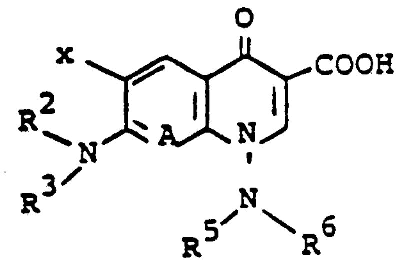

(I)

in which
A denotes either nitrogen or C-$R^1$,
wherein
$R^1$ represents the nitro group or halogen,
$R^2$ and $R^3$ can be identical or different and represent a $C_1$-$C_3$-alkyl radical, or furthermore, together with the nitrogen atom to which they are bonded, can form a 5-membered or 6-membered heterocyclic ring, which can additionally contain the atoms or groups -O-, -S-, -SO-, -$SO_2$- or = N-$R^4$ as a ring member and can optionally be mono-, di- or tri-substituted on the carbon atoms by $C_1$-$C_3$-alkyl, hydroxyl, alkoxy with 1-3 carbon atoms, amino, methylamino or ethylamino, it being possible for a carbon atom in each case to carry only one substituent,
wherein
$R^4$ represents hydrogen, a branched or straight-chain alkyl or alkenyl group which has 1 to 4 carbon atoms and can optionally be substituted by a hydroxyl, alkoxy, alkylmercapto, alkylamino or dialkylamino group with 1 to 3 carbon atoms for an alkyl radical, the cyano group or the alkoxycarbonyl group with 1 to 4 carbon atoms in the alcohol part, or the benzyloxycarbonyl group, a phenylalkyl group which has up to 4 carbon atoms in the aliphatic part and is optionally substituted in the phenyl radical, a phenyl radical which is optionally monosubstituted or disubstituted by hydroxyl, methoxy, chlorine and fluorine, an oxoalkyl radical with up to 6 carbon atoms or a cycloalkylalkyl radical which has up to 6 carbon atoms in the cyclic part and up to 3 carbon atoms in the acyclic part, $R^5$ and $R^6$ can be identical or different and denote hydro-gen or an alkyl group with 1 to 4 carbon atoms, and furthermore X represents hydrogen, the nitro group or halogen, and pharmaceutically useful salts thereof, characterised in that 1-aminoquinolonecarboxylic acids of the general formula II

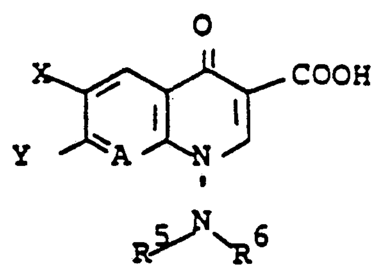

(II)

in which
X, A, $R^5$ and $R^6$ have the abovementioned meaning
and
Y represents halogen,
are reacted with amines of the general formula III

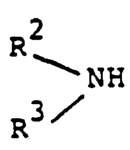

(III)

in which
$R^2$ and $R^3$ have the abovementioned meaning, if appropriate in the presence of acid-binding agents.

10. Use of 1,7-diamino-1,4-dihydro-4-oxo-3-(aza)-quinolinecarboxylic acids of the general formula I

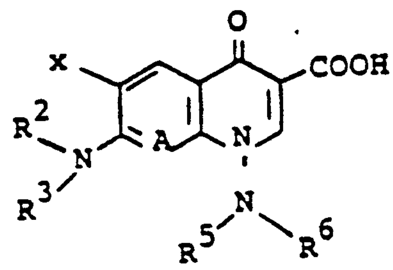

(I)

in which
A denotes either nitrogen or C-$R^1$,
wherein
$R^1$ represents the nitro group or halogen,
$R^2$ and $R^3$ can be identical or different and represent a $C_1$-$C_3$-alkyl radical, or furthermore, together with the nitrogen atom to which they are bonded, can form a 5-membered or 6-membered heterocyclic ring, which can additionally contain the atoms or groups -O-, -S-, -SO-, $-SO_2-$ or = N-$R^4$ as a ring member and can optionally be mono-, di- or tri-substituted on the carbon atoms by $C_1$-$C_3$-alkyl, hydroxyl, alkoxy with 1-3 carbon atoms, amino, methylamino or ethylamino, it being possible for a carbon atom in each case to carry only one substituent,
wherein
$R^4$ represents hydrogen, a branched or straight-chain alkyl or alkenyl group which has 1 to 4 carbon atoms and can optionally be substituted by a hydroxyl, alkoxy, alkylmercapto, alkylamino or dialkylamino group with 1 to 3 carbon atoms for an alkyl radical, the cyano group or the alkoxycarbonyl group with 1 to 4 carbon atoms in the alcohol part, or the benzyloxycarbonyl group, a phenylalkyl group which has up to 4 carbon atoms in the aliphatic part and is optionally substituted in the phenyl radical, a phenyl radical which is optionally monosubstituted or disubstituted by hydroxyl, methoxy, chlorine and fluorine, an oxoalkyl radical with up to 6 carbon atoms or a cycloalkyl-alkyl radical which has up to 6 carbon atoms in the cyclic part and up to 3 carbon atoms in the acyclic part, $R^5$ and $R^6$ can be identical or different and denote hydrogen or an alkyl group with 1 to 4 carbon atoms, and furthermore X represents hydrogen, the nitro group or halogen, and pharmaceutically useful salts thereof, for the preparation of medicaments.

**Revendications**

1. Acides 1,7-diamino-1,4-dihydro-4-oxo-3-(aza)quinoléine-carboxyliques de formule générale I

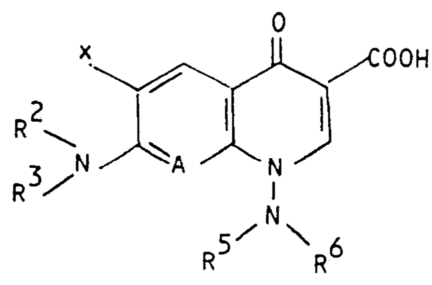

(I)

dans laquelle
A représente l'azote ou un groupe C-$R^1$ dans lequel $R^1$ représente un groupe nitro ou un halogène,
$R^2$ et $R^3$ peuvent avoir des significations identiques ou différentes et représentent chacun un groupe alkyle en $C_1$-$C_3$ ou bien ils forment ensemble et avec l'atome d'azote auquel ils sont reliés un noyau hérérocyclique à 5 ou 6 chaînons qui peut contenir en outre, en tant que chaînons cycliques, les atomes ou groupes -O-, -S-, -SO-, $-SO_2-$ ou bien = N-$R^4$ et qui peut éventuellement être mono- à tri-substitué sur les atomes de carbone par des groupes alkyles en $C_1$-$C_3$, hydroxy, alcoxy en $C_1$-$C_3$, amino, méthylamino ou éthylamino, chaque atome de carbone ne pouvant porter qu'un seul substituant,
$R^4$ représente l'hydrogène, un groupe alkyle ou alcényle à chaîne droite ou ramifiée en $C_1$-$C_4$ qui peut, le cas échéant, être substitué par un groupe hydroxy, alcoxy, alkylmercapto, alkylamino ou dialkylamino contenant 1 à 3 atomes de carbone par groupe alkyle, le groupe cyano, un groupe alcoxycarbonyle contenant 1 à 4 atomes de carbone dans la partie alcool, ou un groupe benzyloxycarbonyle, un groupe phénylalkyle éventuellement substitué dans la partie phényle et contenant jusqu'à 4 atomes de carbone dans la partie aliphatique, un groupe phényle éventuellement mono- ou di-substitué par des groupes

hydroxy, méthoxy, le chlore ou le fluor, un groupe oxoalkyle contenant jusqu'à 6 atomes de carbone ou un groupe cycloalkylalkyle contenant jusqu'à 6 atomes de carbone dans la partie cyclique et jusqu'à 3 atomes de carbone dans la partie acyclique,

et $R^5$ et $R^6$, qui peuvent avoir des significations identiques ou différentes, représentent chacun l'hydrogène ou un groupe alkyle en $C_1$ et $C_4$, et

X représente l'hydrogène, le groupe nitro ou un halogène,

et leurs sels acceptables pour l'usage pharmaceutique.

2. Acides 1,7-diamino-1,4-dihydro-4-oxo-3-(aza)quinoléine-carboxyliques de formule générale I de la revendication 1, dans lesquels X et $R^1$ représentent le fluor ou le chlore, et $R^2$, $R^3$, $R^4$, $R^5$ et $R^6$ ont les significations indiquées ci-dessus.

3. Acides 1,7-diamino-1,4-dihydro-4-oxo-3-(aza)quinoléine-carboxyliques de formule générale I de la revendication 1, dans lesquels A représente l'azote ou C-$R^1$ dans lequel $R^1$ représente le groupe nitro et X, $R^2$, $R^3$, $R^4$, $R^5$ et $R^6$ ont les significations indiquées ci-dessus.

4. L'acide 1-méthylamino-6,8-difluoro-7-(4-méthylpipérazinyl)-1,4-dihydro-4-oxo-3-quinoléine-carboxylique.

5. L'acide 1-méthylamino-6,8-difluoro-7-(1-pipérazinyl)-1,4-dihydro-4-oxo-3-quinoléine-carboxylique.

6. L'acide 1-amino-6,8-difluoro-7-(1-pipérazinyl)-1,4-dihydro-4-oxo-3-quinoléine-carboxylique.

7. L'acide 1-méthylamino-6,8-difluoro-7-(1-pyrrolidinyl)-1,4-dihydro-4-oxo-3-quinoléine-carboxylique.

8. Acides 1,7-diamino-1,4-dihydro-4-oxo-3-(aza)quinoléine-carboxyliques de formule générale I

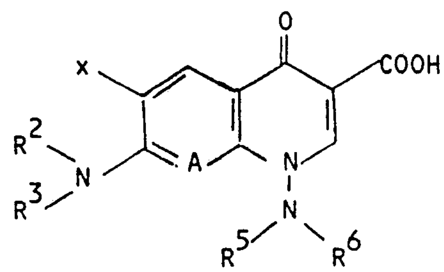

(I)

dans laquelle

A représente l'azote ou un groupe C-$R^1$ dans lequel $R^1$ représente le groupe nitro ou un halogène,

$R^2$ et $R^3$ peuvent avoir des significations identiques ou différentes et représentent chacun un groupe alkyle en $C_1$-$C_3$, mais ils peuvent également former avec l'atome d'azote auquel ils sont reliés un noyau hétérocyclique de 5 ou 6 chaînons qui peut contenir en outre, en tant que chaînons cycliques, les atomes ou groupes -O-, -S-, -SO-, -$SO_2$- ou =N-$R^4$ et qui peut en outre être le cas échéant mono- à tri-substitué sur les atomes de carbone par des groupes alkyle en $C_1$-$C_3$, hydroxy, alcoxy en $C_1$-$C_3$, amino, méthylamino ou éthylamino, chaque atome de carbone ne pouvant porter qu'un seul substituant,

$R^4$ représente l'hydrogène, un groupe alkyle ou alcényle à chaîne droite ou ramifiée en $C_1$-$C_4$ qui peut éventuellement être substitué par un groupe hydroxy, alcoxy, alkylmercapto, alkylamino ou dialkylamino contenant 1 à 3 atomes de carbone par groupe alkyle, le groupe cyano, un groupe alcoxycarbonyle contenant 1 à 4 atomes de carbone dans la partie alcoolique, le groupe benzyloxycarbonyle, un groupe phénylalkyle éventuellement substitué dans la partie phényle et contenant jusqu'à 4 atomes de carbone dans la partie aliphatique, un groupe phényle éventuellement substitué une fois ou deux fois par des groupes hydroxy, méthoxy, le chlore et le fluor, un groupe oxoalkyle contenant jusqu'à 6 atomes de carbone ou un groupe cycloalkylalkyle contenant jusqu'à 6 atomes de carbone dans la partie cyclique et jusqu'à 3 atomes de carbone dans la partie acyclique,

et $R^5$ et $R^6$ peuvent avoir des significations identiques ou différentes et représentent chacun l'hydrogène ou un groupe alkyle en $C_1$-$C_4$, et

X représente l'hydrogène, un groupe nitro ou un halogène, et leurs sels acceptables pour l'usage pharmaceutique pour l'utilisation dans le traitement de maladies bactériennes.

9. Procédé de préparation des acides 1,7-diamino-1,4-dihydro-4-oxo-3-(aza)quinoléine-carboxyliques de formule générale

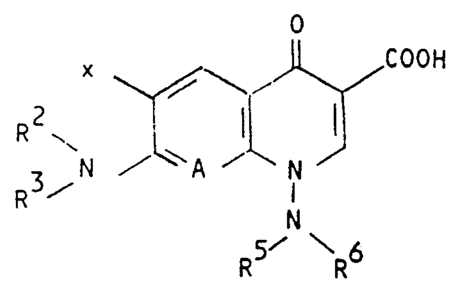

(I)

dans laquelle

A représente l'azote ou un groupe C-$R^1$ dans lequel $R^1$ représente le groupe nitro ou un halogène,

$R^2$ et $R^3$, qui peuvent avoir des significations identiques ou différentes, représentent chacun un groupe alkyle en $C_1$-$C_3$ ou bien ils forment ensemble et avec l'atome d'azote auquel ils sont reliés un noyau hétérocyclique de 5 ou 6 chaînons qui peut contenir en outre, en tant que chaînons cycliques, les atomes ou groupes -O-, -S-, -SO-, $-SO_2-$ ou = N-$R^4$ et qui peut le cas échéant être mono- à tri-substitué sur les atomes de carbone par des groupes alkyle en $C_1$-$C_3$, hydroxy, alcoxy en $C_1$-$C_3$, amino, méthylamino ou éthylamino, chaque atome de carbone ne pouvant porter qu'un seul substituant,

$R^4$ représente l'hydrogène, un groupe alkyle ou alcényle à chaîne droite ou ramifiée en $C_1$-$C_4$ qui peut le cas échéant être substitué par un groupe hydroxy, alcoxy, alkymercapto, alkylamino ou dialkylamino contenant 1 à 3 atomes de carbone par groupe alkyle, le groupe cyano, un groupe alcoxycarbonyle contenant 1 à 4 atomes de carbone dans la partie alcoolique, le groupe benzyloxycarbonyle, un groupe phénylalkyle éventuellement substitué dans la partie phényle et contenant jusqu'à 4 atomes de carbone dans la partie aliphatique, un groupe phényle éventuellement mono- ou di-substitué par des groupes hydroxy, méthoxy, le chlore ou le fluor, un groupe oxoalkyle contenant jusqu'à à 6 atomes de carbone ou un groupe cycloalkylalkyle contenant jusqu'à 6 atomes de carbone dans la partie cyclique et jusqu'à 3 atomes de carbone dans la partie acyclique,

et $R^5$ et $R^6$, qui peuvent avoir des significations identiques ou différentes, représentent chacun l'hydrogène ou un groupe alkyle en $C_1$-$C_4$ et

X représente l'hydrogène, le groupe nitro ou un halogène,

et de leurs sels acceptables pour l'usage pharmaceutique, caractérisé en ce que l'on fait réagir des acides 1-amino-quinolonecarboxyliques de formule générale II

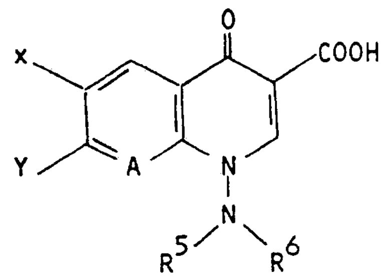

(II)

dans laquelle

X, A et $R^5$, $R^6$ ont les significations indiquées ci-dessus et Y représente un halogène,

avec des amines de formule générale III

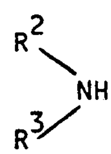

(III)

dans laquelle

$R^2$ et $R^3$ ont les significations indiquées ci-dessus, éventuellement en présence de capteurs d'acides.

10. Utilisation des acides 1,7-diamino-1,4-dihydro-4-oxo-3-(aza)quinoléine-carboxyliques de formule générale I

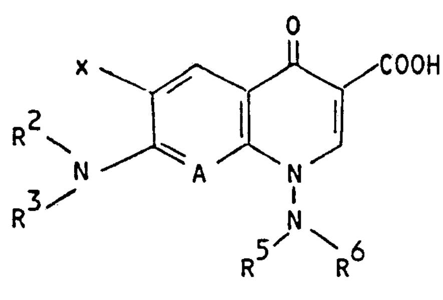

(I)

dans laquelle

A représente l'azote ou le groupe C-$R^1$ dans lequel $R^1$ représente le groupe nitro ou un halogène,

$R^2$ et $R^3$, qui peuvent avoir des significations identiques ou différentes, représentent chacun un groupe alkyle en $C_1$-$C_3$ ou peuvent former ensemble et avec l'atome d'azote auquel ils sont reliés un noyau hétérocyclique à 5 ou 6 chaînons qui peut contenir en outre, en tant que chaînons cycliques, les atomes ou groupes -O-, -S-, -SO-, $-SO_2-$ ou = N-$R^4$ et qui peut le cas échéant être mono- à tri-substitué sur les atomes de carbone par des groupes alkyle en $C_1$-$C_3$, hydroxy, alcoxy en $C_1$-$C_3$, amino, méthylamino ou éthylamino, chaque atome de carbone ne pouvant porter qu'un seul substituant,

$R^4$ représente l'hydrogène, un groupe alkyle ou alcényle à chaîne droite ou ramifiée en $C_1$-$C_4$ qui peut éventuellement être substitué par un groupe hydroxy, alcoxy, alkylmercapto, alkylamino ou dialkylamino contenant 1 à 3 atomes de carbone par groupe alkyle, le groupe cyano, un groupe alcoxycarbonyle contenant 1 à 4 atomes de carbone dans la partie alcoolique, le groupe benzyloxycarbonyle, un groupe phénylalkyle éventuellement substitué dans la partie phényle et contenant jusqu'à 4 atomes de carbone dans la partie aliphatique, un groupe phényle éventuellement mono- ou di-substitué par des groupes hydroxy, méthoxy, le chlore et le fluor, un groupe oxoalkyle contenant jusqu'à 6 atomes de carbone ou un groupe cycloalkylalkyle contenant jusqu'à 6 atomes de carbone dans la partie cyclique et jusqu'à 3 atomes de carbone dans la partie acyclique,

et $R^5$ et $R^6$, ayant des significations identiques ou différentes, représentent chacun l'hydrogène ou un groupe alkyle en $C_1$-$C_4$,

et X représente l'hydrogène, le groupe nitro ou un halogène, et de leurs sels acceptables pour l'usage pharmaceutique pour la préparation de médicaments.